# EUROPEAN PATENT APPLICATION

(11) **EP 4 796 204 A1**
(43) Date of publication of application: **26.08.2026**
(21) Application number: 26159645.6
(22) Date of filing: 19.02.2026
(51) Int. Cl.: B01D 53/04, B01D 53/26

(54) **AIR QUALITY MODULE AND TOOLBOX CONTAINING SAME**

(30) Priority: 21.02.2025 GB 202502546
(71) Applicant: Shone, Jonathan, Northwich CW8 4YP (GB)
(72) Inventor: Shone, Jonathan, Northwich CW8 4YP (GB)
(74) Representative: Prichard, Leslie Stephen

(57) **Abstract**

The present invention relates to an air quality module, comprising:
a rigid housing for containing a moisture-controlling and/or air-purifying and/or corrosion-inhibiting material;
an air-permeable region in the housing to allow air circulation with the moisture-controlling and/or air-purifying and/or corrosion-inhibiting material; and
connecting means enabling placement and retention of the module within a toolbox.

## Description

### TECHNICAL FIELD OF THE INVENTION

This invention relates to an air quality module and toolbox containing same. More particularly, this invention relates to a removable, rechargeable air quality module that can be placed into a toolbox to improve air quality, prolong the life of tools and other contents, and provide a modular and efficient solution for maintaining optimal storage conditions.

### BACKGROUND

The proper storage of tools, equipment and/or other assets often requires maintaining a controlled environment to prevent damage caused by moisture, corrosion or odours. Conventional solutions include desiccant bags, silica gel packets or solid silica sand blocks for moisture management. However, these are often inconvenient to handle, non-rechargeable or lack modularity.

For example, desiccant bags or similar materials are typically loose and must be replaced frequently. While some products allow for recharging in an oven or microwave, they are not designed to be easily handled, stored or re-used efficiently. Existing systems also fail to provide a robust modular design that optimises airflow, packing efficiency or ease of recharging or replacement.

It is an object of the present invention to provide an air quality module and toolbox containing same, which overcomes or reduces the drawbacks associated with known solutions of this type. It is a further object of the present invention to provide a modular, rechargeable and user-friendly solution for air quality improvement. It is a further object of the present invention to provide a mechanism for retaining the air quality module securely within a toolbox for optimal performance.

### SUMMARY OF THE INVENTION

The present invention is described herein and in the claims.

According to the present invention there is provided an air quality module, comprising:
a rigid housing for containing a moisture-controlling and/or air-purifying and/or corrosion-inhibiting material;
an air-permeable region in the housing to allow air circulation with the moisture-controlling and/or air-purifying and/or corrosion-inhibiting material; and
connecting means enabling placement and retention of the module within a toolbox.

An advantage of the present invention is that the air quality module is designed for ease of handling, recharging and replacement, addressing limitations of conventional desiccant bags or loose materials.

In use, the air-permeable region may be selected from the group consisting one or more of the following: apertures, permeable membranes, porous materials or other structures.

Preferably, the air quality module may further comprise a visual indicator to signal when the module requires recharging.

Further preferably, the connecting means is receivable in a retaining means or recess disposed in, or forming part of, the toolbox.

In use, the air-purifying material may comprise activated charcoal.

Preferably, the corrosion-inhibiting material comprises a volatile corrosion inhibitor to prevent rust on stored items.

In use, the volatile corrosion inhibitor may be provided as a Vapour Phase Corrosion Inhibitor foam sheet or a Migrating Corrosion Inhibitor material, or configured as a film, coating or capsule.

Further preferably, the moisture-controlling material is a desiccant.

In use, the desiccant may be selected from the group consisting one or more of the following: bentonite and/or montmorillonite clays, silica gel and/or sand, calcium chloride, molecular sieves.

Preferably, the visual indicator is a colour change indicator.

Further preferably, the housing having two complementary halves that form a chamber for containing the moisture-controlling and/or air-purifying and/or corrosion-inhibiting material.

In use, the housing may comprise a snap-fit connection between the two halves.

Preferably, the housing is formed from two or more components that are assemblable to form the housing.

Further preferably, the components of the housing are fastened by bonding, welding, or other mechanical fixing and fasteners.

Preferably, the toolbox is a cabinet or enclosure.

Also according to the present invention there is provided a toolbox, comprising:
at least one compartment configured to contain tools or accessories; and
a removable module containing a moisture-controlling element being disposed, or
received, within the compartment to regulate humidity levels.

Preferably, the toolbox further comprising a retaining clip to hold the module securely.

Further preferably, the retaining clip is integrated within the toolbox, or is secured thereto via threaded fasteners or adhesive or the like.

In use, the air quality module may be retained within a lattice-like structure or ribs disposed within the toolbox.

Preferably, the removable module includes a visual indicator for signalling moisture-recharging requirements.

Further preferably, the toolbox may comprise:
a corrosion-inhibiting component to prevent rust on stored items; and
an air-purifying feature designed to eliminate odours and/or pollutants.

In use, the moisture-controlling element may comprise desiccant material.

Preferably, the moisture-controlling element is positionable within the lid of the toolbox.

Further preferably, the moisture-controlling element is configured to be rechargeable using a microwave or similar heat source or by exposure to solar radiation and/or through the convective assistance of wind, or forced convection, or by recharging through exposure to other parts of the electromagnetic spectrum, including those in the infrared or ultraviolet wavelengths.

In use, the removable module may be formed from a microwave-safe plastics material selected from the group consisting, but not limited to, any of the following: Polycarbonate (PC), Polypropylene (PP), High-Density Polyethylene (HDPE), Polyethylene Terephthalate (PET), Polyethylene (PE), resins or blends thereof.

It is believed that an air quality module and toolbox containing the same in accordance with the present invention at least addresses the problems outlined above.

It will be obvious to those skilled in the art that variations of the present invention are possible, and it is intended that the present invention may be used other than as specifically described herein.

### BRIEF DESCRIPTION OF THE DRA WINGS

The present invention will now be described by way of example only, and with reference to the accompanying drawings, in which:
Figure 1 is a perspective view from the side and above of an air quality module that, in use, is contained within a toolbox or the like;
Figure 2 shows a perspective view from the side and above of a retention means for retaining the air quality module within a toolbox;
Figure 3 illustrates an exploded perspective view from the side and above, and showing how the retention means is configured to slidably accommodate the air quality module, and thereby enabling its retention against a section of a toolbox;
Figures 4 and 5 show how the air quality module of the present invention can be contained within a toolbox or the like, with Figure 4 showing the air quality module retained within the main compartment of the toolbox via the retention means shown in

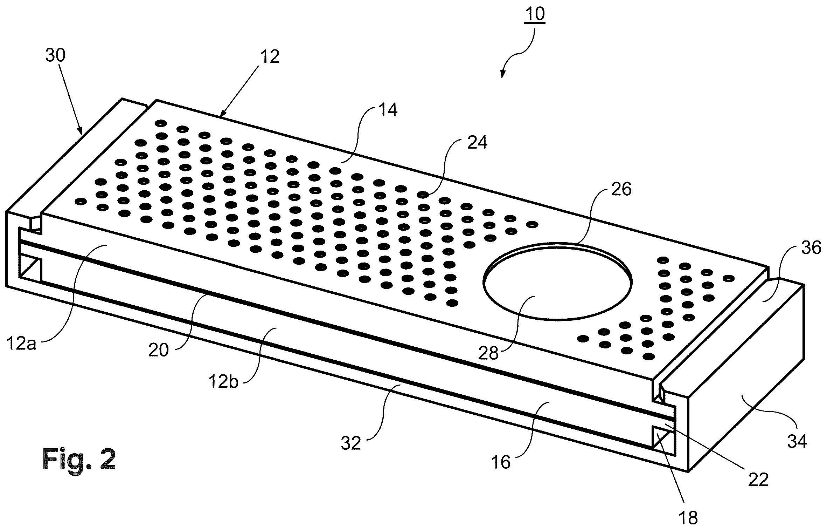

Figures 2 and 3, and Figure 5 showing an alternative embodiment of the present invention whereby the air quality module is retained by way of a transition fit within in-moulded slots or ribs formed in the lid of the toolbox; and

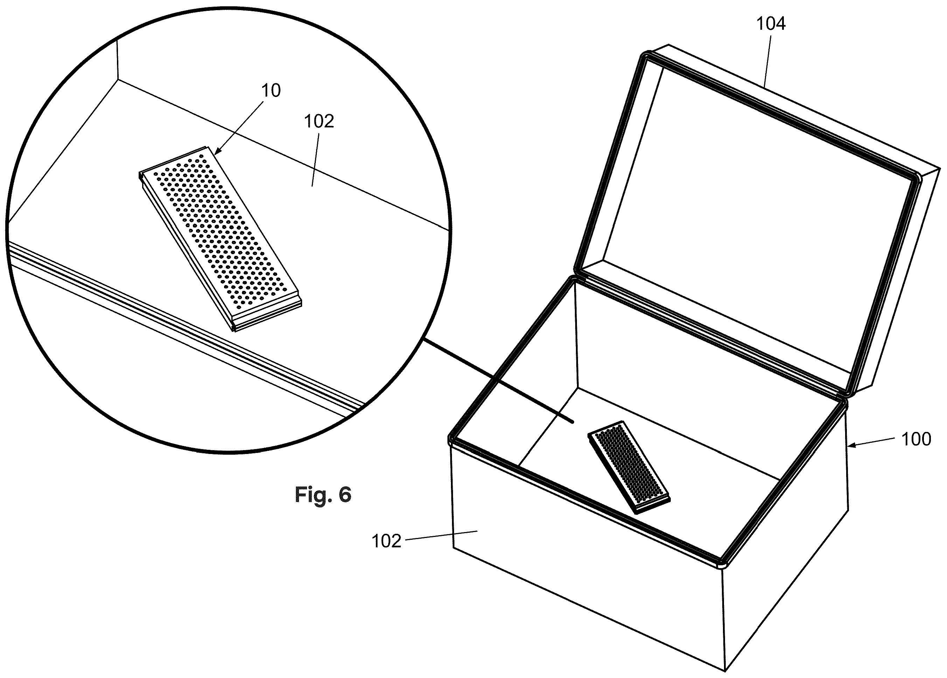

Figure 6 illustrates that the air quality module of the present invention can be used independently, without the need for a retention means, and can be placed directly into a compartment of the toolbox.

### DETAILED DESCRIPTION OF THE PREFERRED EMBODIMENTS

The present invention has adopted the approach of utilising a modular air quality module and a toolbox containing same that can be easily recharged and replaced. Advantageously, the present invention provides a robust and user-friendly solution for maintaining optimal air quality and preventing damage to stored items. Further advantageously, the present invention also provides a means of securely retaining the module within a toolbox for controlled and efficient air circulation.

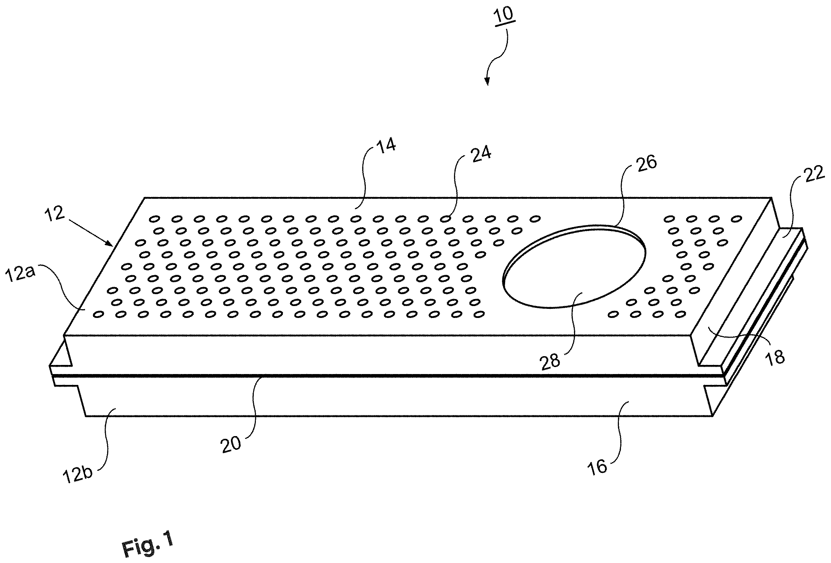

Referring now to the drawings, an air quality module 10 according to the present invention is illustrated in Figures 1 to 6. In a preferred embodiment of the invention, the air quality module 10 is for fitment into a toolbox 100, as will be described in further detail below.

The air quality module 10 is a rigid body 12 which consists of two complementary-shaped halves 12a, 12b that together enclose a desiccant or the like for air quality management. As illustrated in Figure 1, the air quality module 10 has an elongated and generally rectangular-shape when viewed from above in plan view. However, this specific configuration is not intended to be restrictive, as the present invention can be realised in a wide variety of shapes and sizes to suit different applications.

Each half 12a, 12b of the rigid body 12 is formed as a largely planar tray-like body, and having a generally flat base wall 14 with a pair of side walls 16 which extend upwardly along each longitudinal edge of the base wall 14. A pair of end walls 18 also extend upwardly from the shorter sides of the base wall 14 such that the base wall 14, side walls 16 and end walls 18 in combination define an open chamber which can receive a desiccant material, to be described later.

Away from the base wall 14, the side walls 16 and end walls 18 in combination forms a rim or lip 20 that is in a plane substantially parallel to the base wall 14. In use, one half 12a of the module 10 is nested against an upturned complementary half 12b, forming a sealed pocket or chamber around the rim 20 that securely retains the desiccant entirely within.

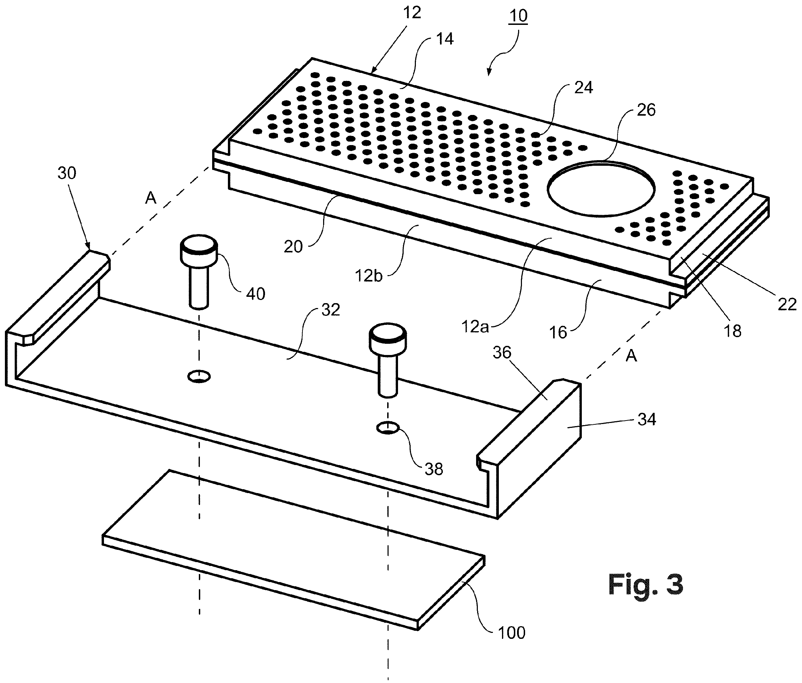

As perhaps best shown in Figures 1 and 3, disposed along each of the end walls 18 is a continuous protrusion or lug 22 that is in a plane substantially parallel to the base wall 14. Again, when mated, the protrusion 22 disposed along both of the end walls 18 of the first half 12a mates with the protrusion 22 disposed along both of the end walls 18 of an upturned complementary half 12b. The protrusions 22 at the ends of the rigid body 12 can, in use, be slidably received in a retaining clip 30 for quick and secure retention of the air quality module 10 within a toolbox 100, as described in further detail with respect to Figures 2 to 4.

The skilled person will understand that the two halves 12a, 12b when brought together can include a snap-fit connection or other means of fastening as would be known to a person skilled in the art. The sealed pocket or chamber formed around the rim 20 securely retains a desiccant material entirely within.

One or both halves 12a, 12b of the air quality module 10 feature a series of apertures 24 in the base wall 14, allowing the air inside the toolbox 100 to interact with the desiccant material. Although not shown in the drawings, instead of apertures 24 it is entirely within the scope of the invention to include permeable membranes or other porous materials or other structures that achieve the same function of allowing air interaction with the with the desiccant material.

One or more larger apertures 26 may also be disposed in the base wall 14 of one or both halves 12a, 12b of the air quality module 10. Such aperture 26 enables a visual indicator to show when the desiccant needs to be recharged. In a preferred embodiment, the aperture 26 contains a colour change indicator 28 being is a printed paper indicator that changes from one colour to another when the desiccant needs to be recharged, or an indicator strip, which changes colour when specific humidity levels are reached and the desiccant requires recharging. Alternatively, the colour change indicator 28 can be a transparent window that enables a user to directly view desiccant beads that themselves include a colour indicating material depending upon their humidity.

In the example shown in the accompanying drawings, half 12a is depicted with these apertures 24, 26 while half 12b may remain entirely blank.

The skilled person will understand that the rigid body 12, which is illustratively shown in Figures 1 to 3 as consisting of two complementary-shaped halves 12a, 12b, can be assembled from more than two components that together form the rigid body 12. As mentioned above, other means of fastening the components of the rigid body 12 are entirely possible. These can include assembly by bonding, welding or other mechanical fixing and fasteners. The above list is no way intended to be limiting and exhaustive. One or more of the assembled air quality modules 10 can, in one embodiment of the invention, simply be placed into the enclosed volume of a toolbox 100 or the like, as shown in Figure 6, to improve air quality, prolong the life of tools and other contents, and provide an efficient solution for maintaining optimal storage conditions. The module 10 is designed to be removed from the toolbox 100 and is rechargeable using a microwave or similar heat source, as required.

Recharging of the module 10 may also be possible by direct solar radiation and/or through the convective assistance of wind, or forced convection. Recharging by exposure to other parts of the electromagnetic spectrum, including those in the infrared or ultraviolet ranges, would also be envisaged.

Desiccants are a class of materials that attract and hold moisture from the air, primarily through adsorption, whereby moisture adheres to the surface rather than being absorbed into the material. Different types of desiccants are entirely possible within the scope of the invention including: bentonite and montmorillonite clays, silica gel, calcium chloride and molecular sieves. Clay desiccants are cost-effective and ideal for moderate humidity, adsorbing up to 30% of their weight. Silica gel, known for its non-toxic and non-flammable properties, adsorbs up to 40% of its weight and is effective at higher temperatures. Calcium chloride has a high moisture-adsorbing capacity, adsorbing up to 200% of its weight, and works across a wide temperature range. Molecular sieves, with their specialised structure, selectively adsorb water molecules and are particularly effective at higher temperatures. Each type has its unique benefits depending on the specific moisture control needs. The above list is no way intended to be limiting and exhaustive.

In addition to using a moisture-controlling desiccant within a rigid housing 12, the module 10 can also be alternately configured as a solid block of material utilising natural silica sand. The solid block structure having a porous structure, enabling strong moisture absorption and effective regeneration capabilities. It is envisaged that such formulation can be injection-moulded.

The module 10 may also incorporate additional elements to enhance its functionality. These could include a Volatile Corrosion Inhibitor (VCI) to provide extra protection against rust, complementing the desiccant's moisture control functionality. The VCI could be provided as a Vapour Phase Corrosion Inhibitor (VpCI) foam sheet or a Migrating Corrosion Inhibitor (MCI) material, or provided as a film, coating or capsule. This is in no way intended to be limiting and exhaustive. Additionally, activated charcoal can be included to effectively eliminate odours and air pollutants.

In other embodiments of the invention, the air quality module 10 can be retained securely within a toolbox 100 by way of a retaining clip 30, that is best illustrated in Figures 2 and 3. The retaining clip 30 is elongate and is generally rectangular in shape, when in plan view from above. However, this specific configuration is not intended to be restrictive, as the present invention can be realised in a wide variety of shapes and sizes to suit different applications.

The retaining clip 30 has a generally flat base 32 with a pair of end walls 34 which extend upwardly from the shorter sides of the base wall 32. Disposed inwardly along each of the end walls 34 is a continuous projection 36 that is in a plane substantially parallel to the base wall 32, such that the base wall 32, end walls 34 and projections 36 in combination define a generally C-shaped opening to receive each protrusion 22 disposed at each end 18 of the module 10.

It is clear from the view of Figures 2 and 3 that the projections 36 which are only connected to the end walls 34, are able to be urged apart slightly and the module 10 can be inserted therebetween through the C-shaped opening formed by the base wall 32, end walls 34 and projections 36 and which then retains the module 10 in place via a snap-fit or slide connection. In use, the air quality modules 10 is slid along the dotted line A in Figure 3 for secure attachment.

In order that the air quality module 10 might be clipped or snap-fitted into the clip 30, the gap or opening between the opposite edges of the projections 36 of the clip 30 must not be too small to enable the module 10 to be forced into the clip 30 without causing damage to either component. After sliding the air quality module 10 in place along dotted line A, it is a transition fit between the projections 36 of the clip 30 and the arms 22 of the module 10 that generates enough purchase to hold the module 10 safely in position. To remove the air quality module 10 for recharging or replacement, the same steps are followed in reverse.

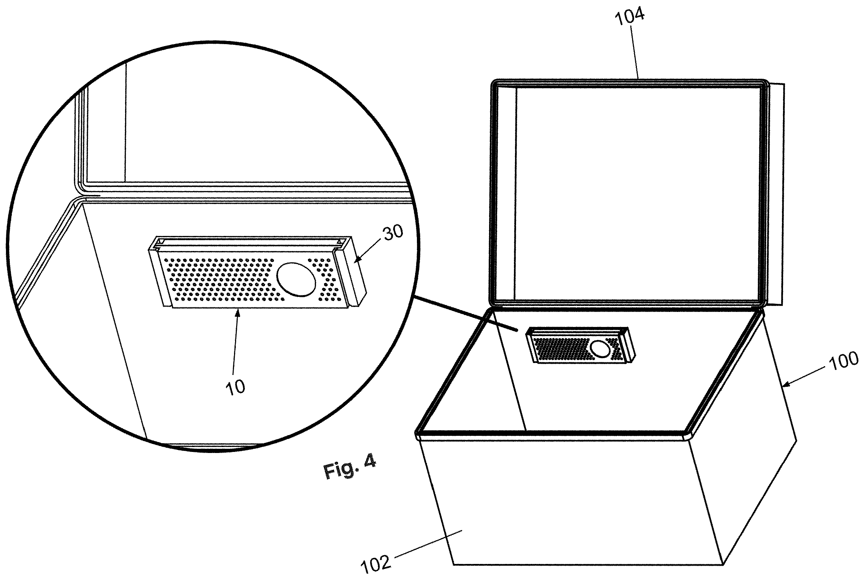

Also as shown in Figure 3, apertures 38 in the clip base 32 allow it to be secured to the toolbox 100 via threaded fasteners 40 or alternative attachment means such as adhesives or the like are envisaged. The module 10 can therefore be placed directly into a toolbox 100 or retained via a clip 30, as shown in Figures 3 and 4.

Equally, the retaining clip 30 can form part of the toolbox 100 itself, by in-moulding such retaining arm within the body 102 and/or lid 104 of the toolbox.

The assembled module 10 can be placed directly into a toolbox 100 or retained via a clip 30, as shown in Figures 2 to 4.

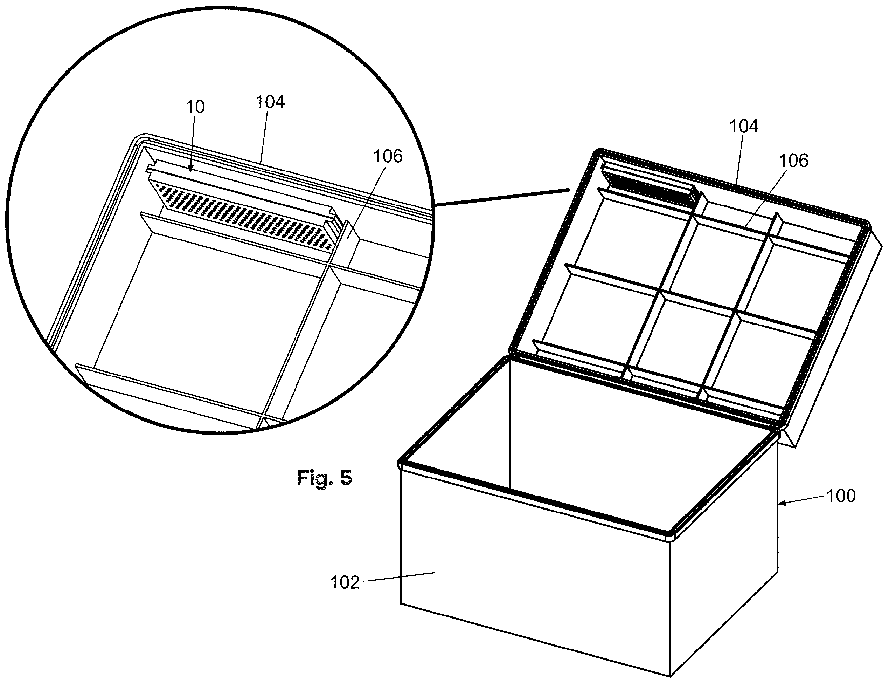

Figure 5 illustrates an alternative method for securing the air quality module 10 within a toolbox 100, using ribs 106 located inside the lid 104 of the toolbox 100 the form recesses. The spacing between the ribs 106 is designed to hold the projections 22 of the module 10 in place, either by abutment between pairs of ribs 106 or between a rib 106 and the side of the lid 104, creating a transition fit. The skilled person will understand that multiple control modules 10 can be securely retained in the toolbox 100 using this method.

In a preferred embodiment, the components forming the module 10 and toolbox 100 containing same are formed from a polymer such as such as Polycarbonate (PC), Polypropylene (PP), High-Density Polyethylene (HDPE), Polyethylene Terephthalate (PET) or Polyethylene (PE) or blends thereof. Equally, the skilled person will appreciate that the components forming the module 10 and toolbox 100 containing same can be formed from any number of synthetic plastics, such as a thermoplastic or thermoset material. The above plastics are microwave-safe and do not emanate any toxic fumes or deform when heated with microwaves. The above list is no way intended to be limiting or exhaustive.

The components forming the module 10 and toolbox 100 containing same can be manufactured using techniques such as injection moulding, blow moulding, vacuum forming, rotational moulding, compression moulding, rim moulding, powder impression moulding or any other form of plastics or rubber manufacture, as additive manufacturing or 3D printing.

The components forming the module 10 and toolbox 100 containing same are designed to be UV stable, waterproof and reusable, ensuring durability and resilience in various environmental conditions. Additionally, they are manufactured to be waste-free, contributing significantly to the module's 10 sustainability by minimising environmental impact and promoting long-term usability.

Therefore, an air quality module 10 and toolbox 100 containing same is provided, and which provides the advantages outlined above.

When used in this specification and claims, the terms "comprises" and "comprising" and variations thereof mean that the specified features, steps or integers are included. The terms are not to be interpreted to exclude the presence of other features, steps or components. The singular forms "a", "an" and "the" are intended to include the plural forms as well, unless the context clearly indicates otherwise. It will be further understood that the terms "includes" and/or "including" when used herein, specify the presence of stated features, steps, operations, elements, and/or components, but do not preclude the presence or addition of one or more other features, steps, operations, elements, components, and/or groups thereof.

The features disclosed in the foregoing description, or the following claims, or the accompanying drawings, expressed in their specific forms or in terms of a means for performing the disclosed function, or a method or process for attaining the disclosed result, as appropriate, separately, or in any combination of such features, can be utilised for realising the invention in diverse forms thereof.

The invention is not intended to be limited to the details of the embodiments described herein, which are described by way of example only. It will be understood that features described in relation to any particular embodiment can be featured in combination with other embodiments.

It is contemplated by the inventor that various substitutions, alterations and modifications may be made to the invention without departing from the spirit and scope of the invention as defined by the claims.

### CLAUSES

The following clauses define preferred embodiments of the invention.
1. An air quality module, comprising:
   a rigid housing for containing a moisture-controlling and/or air-purifying and/or corrosion-inhibiting material;
   an air-permeable region in the housing to allow air circulation with the moisture-controlling and/or air-purifying and/or corrosion-inhibiting material; and
   connecting means enabling placement and retention of the module within a toolbox.
2. The air quality module of clause 1, wherein the air-permeable region is selected from the group consisting one or more of the following: apertures, permeable membranes, porous materials or other structures.
3. The air quality module of clause 1, further comprising a visual indicator to signal when the module requires recharging.
4. The air quality module of clause 1, wherein the connecting means is receivable in a retaining means or recess disposed in, or forming part of, the toolbox.
5. The air quality module of clause 1, wherein the air-purifying material comprises activated charcoal.
6. The air quality module of clause 1, wherein the corrosion-inhibiting material comprises a volatile corrosion inhibitor to prevent rust on stored items.
7. The air quality module of clause 7, wherein the volatile corrosion inhibitor is provided as a Vapour Phase Corrosion Inhibitor foam sheet or a Migrating Corrosion Inhibitor material, or configured as a film, coating or capsule.
8. The air quality module of clause 1, wherein the moisture-controlling material is a desiccant.
9. The air quality module of clause 8, wherein the desiccant may be selected from the group consisting one or more of the following: bentonite and/or montmorillonite clays, silica gel and/or sand, calcium chloride, molecular sieves.
10. The air quality module of clause 3, wherein the visual indicator is a colour change indicator.
11. The air quality module of clause 1, wherein the housing having two complementary halves that form a chamber for containing the moisture-controlling and/or air-purifying and/or corrosion-inhibiting material.
12. The air quality module of clause 11, wherein the housing comprises a snap-fit connection between the two halves.
13. The air quality module of clause 1, wherein the housing is formed from two or more components that are assemblable to form the housing.
14. The air quality module of clause 13, wherein the components of the housing are fastened by bonding, welding, or other mechanical fixing and fasteners.
15. The air quality module of clause 1, wherein the toolbox is a cabinet or enclosure.
16. A toolbox, comprising:
   at least one compartment configured to contain tools or accessories; and
   a removable module containing a moisture-controlling element being disposed, or
   received, within the compartment to regulate humidity levels.
17. The toolbox of clause 16, further comprising a retaining clip to hold the module securely.
18. The toolbox of clause 17, wherein the retaining clip is integrated within the toolbox, or is secured thereto via threaded fasteners or adhesive or the like.
19. The toolbox of clause 16, wherein the air quality module is retained within a lattice-like structure or ribs disposed within the toolbox.
20. The toolbox of clause 16, wherein the removable module includes a visual indicator for signalling moisture-recharging requirements.
21. The toolbox of clause 16, further comprising:
   a corrosion-inhibiting component to prevent rust on stored items; and
   an air-purifying feature designed to eliminate odours and/or pollutants.
22. The toolbox of clause 16, wherein the moisture-controlling element comprises desiccant material.
23. The toolbox of clause 16, wherein the moisture-controlling element is positionable within the lid of the toolbox.
24. The toolbox of clause 16, wherein the moisture-controlling element is configured to be rechargeable using a microwave or similar heat source or by exposure to solar radiation and/or through the convective assistance of wind, or forced convection, or by recharging through exposure to other parts of the electromagnetic spectrum, including those in the infrared or ultraviolet wavelengths.
25. The toolbox of clause 16, wherein the removable module is formed from a microwave-safe plastics material selected from the group consisting, but not limited to, any of the following: Polycarbonate (PC), Polypropylene (PP), High-Density Polyethylene (HDPE), Polyethylene Terephthalate (PET), Polyethylene (PE), resins or blends thereof.

## Claims

1. An air quality module, comprising:
a rigid housing for containing a moisture-controlling and/or air-purifying and/or corrosion-inhibiting material;
an air-permeable region in the housing to allow air circulation with the moisture-controlling and/or air-purifying and/or corrosion-inhibiting material; and
connecting means enabling placement and retention of the module within a toolbox.

2. The air quality module of claim 1, wherein the air-permeable region is selected from the group consisting one or more of the following: apertures, permeable membranes, porous materials or other structures.

3. The air quality module of claim 1, further comprising a visual indicator to signal when the module requires recharging,
and wherein the visual indicator is a colour change indicator.

4. The air quality module of claim 1, wherein the connecting means is receivable in a retaining means or recess disposed in, or forming part of, the toolbox,
and wherein the toolbox is a cabinet or enclosure.

5. The air quality module of claim 1, wherein the air-purifying material comprises activated charcoal.

6. The air quality module of claim 1, wherein the corrosion-inhibiting material comprises a volatile corrosion inhibitor to prevent rust on stored items,
and wherein the volatile corrosion inhibitor is provided as a Vapour Phase Corrosion Inhibitor foam sheet or a Migrating Corrosion Inhibitor material, or configured as a film, coating or capsule.

7. The air quality module of claim 1, wherein the moisture-controlling material is a desiccant, wherein the desiccant may be selected from the group consisting one or more of the following: bentonite and/or montmorillonite clays, silica gel and/or sand, calcium chloride, molecular sieves.

8. The air quality module of claim 1, wherein the housing having two complementary halves that form a chamber for containing the moisture-controlling and/or air-purifying and/or corrosion-inhibiting material,
and wherein the housing comprises a snap-fit connection between the two halves.

9. The air quality module of claim 1, wherein the housing is formed from two or more components that are assemblable to form the housing,
and wherein the components of the housing are fastened by bonding, welding, or other mechanical fixing and fasteners.

10. A toolbox, comprising:
at least one compartment configured to contain tools or accessories; and
a removable module containing a moisture-controlling element being disposed, or
received, within the compartment to regulate humidity levels.

11. The toolbox of claim 10, further comprising a retaining clip to hold the module securely,
and wherein the retaining clip is integrated within the toolbox, or is secured thereto via threaded fasteners or adhesive or the like.

12. The toolbox of claim 10, wherein the air quality module is retained within a lattice-like structure or ribs disposed within the toolbox.

13. The toolbox of claim 10, wherein the removable module includes a visual indicator for signalling moisture-recharging requirements,
and wherein the moisture-controlling element is configured to be rechargeable using a microwave or similar heat source or by exposure to solar radiation and/or through the convective assistance of wind, or forced convection, or by recharging through exposure to other parts of the electromagnetic spectrum, including those in the infrared or ultraviolet wavelengths.

14. The toolbox of claim 10, further comprising:
a corrosion-inhibiting component to prevent rust on stored items; and
an air-purifying feature designed to eliminate odours and/or pollutants.

15. The toolbox of claim 10, wherein the moisture-controlling element comprises desiccant material,
and wherein the moisture-controlling element is positionable within the lid of the toolbox,
and wherein the moisture-controlling element is configured to be rechargeable using a microwave or similar heat source or by exposure to solar radiation and/or through the convective assistance of wind, or forced convection, or by recharging through exposure to other parts of the electromagnetic spectrum, including those in the infrared or ultraviolet wavelengths.
